Europäisches Patentamt

European Patent Office

Office européen des brevets

(11) Numéro de publication: **0 308 315 B1**

## (12) FASCICULE DE BREVET EUROPEEN

(45) Date de publication de fascicule du brevet: **15.12.93** (51) Int. Cl.⁵: **G06F 15/353**

(21) Numéro de dépôt: **88402299.7**

(22) Date de dépôt: **13.09.88**

(54) **Procédé d'interpolation.**

(30) Priorité: **16.09.87 FR 8712814**

(43) Date de publication de la demande:
**22.03.89 Bulletin 89/12**

(45) Mention de la délivrance du brevet:
**15.12.93 Bulletin 93/50**

(84) Etats contractants désignés:
**DE GB NL**

(56) Documents cités:
**EP-A- 0 070 677**
**GB-A- 2 026 811**
**US-A- 4 446 529**

**TECHNISCHE MESSEN, vol. 51, no. 5, mai 1984, pages 176-186, München, DE; H. JANO-CHA: "Digitales Speicherverfahren zur auto-matischen Linearisierung von Kennlinien der Form u = g(x,y)"**

**HEWLETT-PACKARD JOURNAL, vol. 34, no. 10, octobre 1983, pages 30-34, Amstelveen NL; S.C. LEAVITT et al.: "A scan conversion algorithm for displaying ultrasound images"**

(73) Titulaire: **COMMISSARIAT A L'ENERGIE ATO-MIOUE**
**31/33, rue de la Fédération**
**F-75015 Paris Cédex 15(FR)**

(72) Inventeur: **Tararine, Michel**
**6, rue du Four**
**F-92330 Sceaux(FR)**
Inventeur: **Thévenin, Bernard**
**1 Chemin La Bourrelière**
**F-38120 Saint Egrève(FR)**

(74) Mandataire: **Mongrédien, André et al**
**c/o BREVATOME**
**25, rue de Ponthieu**
**F-75008 Paris (FR)**

EP 0 308 315 B1

## Description

La présente invention à pour objet un procédé d'interpolation de la valeur d'un point dans une maille à N sommets. L'interpolation étudiée est de préférence de type bilinéaire encore que l'invention s'applique à des cas où elle ne serait ni linéaire ni même double mais plutôt multiple. Dans l'exemple décrit la maille comporte quatre sommets mais dans certaines applications, en particulier dans des applications d'imagerie en représentation d'objets à trois dimensions, la maille pourrait comporter un nombre différent de sommets : par exemple trois, six ou huit sommets. Le procédé d'interpolation de l'invention trouve une application dans le domaine médical où il peut être utilisé pour corriger des distorsions d'images acquises avec les gamma-caméras. Il peut aussi être utilisé dans le domaine vidéo où il permet de représenter des images de faible définition, par exemple à 64 X 64 éléments d'image, comme des images haute définition, par exemple à 256 X 256 éléments d'image. Le principal intérêt du procédé d'interpolation de l'invention réside dans la rapidité avec laquelle l'interpolation peut être faite. Cette rapidité autorise le traitement en temps réel en respectant pour visualiser les images les normes habituelles de représentation vidéo télévision.

Les gamma-caméras sont utilisées en médecine nucléaire pour visualiser dans un organe la répartition de molécules marquées par un isotope radioactif que l'on a injecté à un patient. Une gamma-caméra comprend généralement un collimateur pour focaliser les photons gammas émis par le patient, un cristal scintillateur pour transformer les photons gamma en photons lumineux ou scintillations, et un réseau de tubes photo-multiplicateurs qui transforment chacune des scintillations en des impulsions électriques dites contributions électriques de tubes. Elles comportent en outre des circuits électroniques pour produire à partir des contributions électriques fournies par les tubes photo-multiplicateurs, des signaux de coordonnées X et Y du lieu où s'est produite une scintillation, ainsi qu'un signal de validation Z quand l'énergie W de la scintillation appartient à une bande d'énergie prédéterminée.

Cette chaîne de détection est généralement suivie d'un ensemble de visualisation qui peut comporter un oscilloscope cathodique commandé par les signaux de coordonnés X, Y, et par Z pour visualiser par un point lumineux sur l'écran le point d'impact du photon gamma sur le cristal. Cet impact est aussi appelé évènement d'image. L'ensemble de visualisation peut éventuellement comporter un dispositif photographique pour former une image de l'organe observé en intégrant un grand nombre de points lumineux produits sur l'écran cathodique. Il peut par ailleurs comprendre un traitement numérique des images. En particulier l'ensemble de visualisation peut être adapté à la représentation de tomographies de l'organe observé. Pour atteindre ce but on acquiert plusieurs images de cet organe selon une pluralité d'orientations d'observation de la gamma-caméra par rapport à cet organe. Par des traitements du signal, analogues à ceux rencontrés dans les tomodensitomètres, on peut reconstituer des images de coupes des organes examinés.

En autres qualités une gamma-caméra doit posséder une bonne résolution spatiale, c'est à dire la capacité de distinguer des petites sources radioactives rapprochées, une bonne réponse en taux de comptage, c'est à dire la capacité de traiter un grand nombre d'évènements par unité de temps, et une qualité d'image le plus possible indépendante de l'énergie de l'isotope considéré. La résolution spatiale dépend de la précision des calculs des coordonnées X et Y de chacun des évènements d'image. La qualité de l'élaboration de ces coordonnées dépend de la précision des mesures et des lois physiques régissant le fonctionnement des différentes parties de la gamma-caméra. Ainsi l'interaction d'un photon gamma avec le cristal donne naissance à une scintillation lumineuse dont l'intensité décroît expotentiellement avec le temps. Cette scintillation est vue par plusieurs tubes photo-multiplicateurs simultanément. Les photons lumineux composant cette scintillation arrachent des photo-électrons aux photo-cathodes des tubes photo-multiplicateurs. Le nombre des photo-électrons arrachés obéit, pour une scintillation donnée, à la loi statistique de Poisson. Par ailleurs, à énergie constante, la contribution électrique est une fonction sensiblement gaussienne de la distance séparant le centre du tube photo-multiplicateur de la projection du lieu où s'est produite la scintillation. Si la scintillation se produit à l'aplomb du centre du tube, la contribution électrique est maximum. Plus le lieu de la scintillation est éloigné du centre du tube plus la contribution électrique est faible. A titre d'exemple, si une scintillation se produit à l'aplomb de la paroi du tube la contribution électrique de celui-ci est environ réduite de moitié par rapport à la contribution électrique maximum.

Une scintillation est vue par plusieurs tubes photo-multiplicateurs simultanément, généralement 6 à 10 tubes. Aussi, la détermination de l'emplacement de cette scintillation sur le cristal, elle-même représentative du lieu d'émission du photon gamma d'excitation (et donc de l'évènement d'image), peut être obtenue en calculant l'emplacement du barycentre des contributions électriques délivrés par l'ensemble des tubes photo-multiplicateurs excités par cette scintillation. Ce calcul s'effectue simplement, selon le brevet américain ANGER N° 3011057, en injectant les contributions éléctriques au travers d'un jeu de matrices de résistances dont les valeurs des résistances sont fonction des positions des tubes photo-multiplicateurs auxquels elles sont raccordées. Les positions de ces tubes sont repérées par rapport à des axes cartésiens de référence dont le point d'intersection est généralement situé au centre du réseau de tubes. Dans chaque matrice il y a autant de résistances qu'il y a de tubes photo-multiplicateurs dans le réseau de tubes. Chacune des résistances est reliée d'une part à la sortie d'un tube photo-multiplicateur différent et d'autre part à un point commun qui constitue la sortie de la matrice. Ces résistances effectuent ainsi une pondération des contributions électriques de chacun des tubes de photo-multiplicateurs qui les alimentent.

Un des problèmes présentés par les gamma-caméra du type ANGER est qu'elles présentent des distorsions géométriques de l'image liées à la structure de captation de la lumière : cristal scintillateur-tube photomultiplicateur-matrice de barycentration.

Les progrès de la médecine nucléaire, et en particulier les améliorations faites pour recueillir davantage d'informations, et des informations meilleures, à partir des gamma-caméras, par exemple pour la détection de petites tumeurs, conduisent à optimiser la résolution spatiale au détriment de la linéarité caractéristique inhérente à la conception et à la réalisation des caméras. Il en résulte une distorsion spatiale gênante des images. Ces distorsions sont à l'origine de défauts d'uniformité de densité qui peuvent être importants. A titre d'exemple, une contraction de 0,4 mm du rayon de l'image d'une surface circulaire d'un centimètre de rayon conduit à un défaut d'uniformité de 8 %. Ces défauts d'uniformité peuvent être particulièrement gênants en tomographie où l'effet d'amplification des défauts d'uniformité dûs aux procédés de reconstruction peut être supérieur à un facteur 10.

Dans l'état de la technique on a tenté de corriger les effets de cette distorsion. Le principe général de la correction est le suivant. On effectue la mesure d'une image d'une mire régulière placée entre une source radioactive d'émission uniforme et la gamma-caméra. Si la gamma-caméra était parfaite, l'image représentée devrait correspondre à la répartition régulière des trous ou des fentes de la mire. La mesure effectuée montre en fait des irrégularités provenant des effets de distorsion spatiales. On peut cependant utiliser dans l'image ainsi acquise la connaissance de la distorsion, mesurée en comparant l'image acquise à la répartition théorique à laquelle on aurait dû aboutir, pour effectuer les corrections des images ultérieurement acquises avec la même gamma-caméra.

Dans un état de la technique, constitué par le brevet européen N° 0021.366, un calculateur élabore une matrice de correction de 64 X 64 nouvelles coordonnées U et V d'une image non distordue correspondant à un même nombre de coordonnées X et Y de l'image distordue de la mire. En fonctionnement de correction, les coordonnées acquises X et Y des évènements d'images sont codées sur 12 bits. Les 6 bits de poids fort de X et Y adressent la mémoire des U et V. Puis une interpolation linéaire est effectuée en utilisant les 6 bits de poids faible de X et Y. On utilise ainsi le décalage d'un évènement d'image à placer par rapport aux quatre angles de la maille élémentaire U, V, de l'image corrigée dans laquelle l'élément d'image doit finalement se trouver. Compte tenu d'un champ image d'environ 400 mm de diamètre, une codification sur 12 bits des coordonnées des évènements d'image devrait normalement conduire à une définition d'image de l'ordre de 0,1 mm.

On a pu montrer que cette définition ne pouvait être obtenue réellement que si la matrice de correction était en fait une matrice de 128 X 128 mailles et si donc on utilisait 7 bits de poids fort pour déterminer dans quelle maille de l'image corrigée doit se trouver l'évènement d'image à placer. Cette détermination est équivalente à attribuer des corrections de coordonnées aux sommets des 128 X 128 mailles de l'image acquise. Ceci étant, cette différence n'influe pas dans le principe de la présente invention. En effet la réprésentation des images avec 128 X 128 évènements d'image est aussi trop grossière. Il est nécessaire d'atteindre une résolution plus fine. Compte tenu du fait que les coordonnées acquises des évènements d'image (de l'image distordue) sont données sur 12 bits, il reste 5 bits pour faire une interpolation de la position vraie d'un évènement d'image dans la maille corrigée à laquelle on vient de déterminer qu'il doit appartenir. On calcule par interpolation le déplacement à appliquer à l'évènement, à partir des corrections de coordonnées à appliquer aux sommets des mailles. Si on appelle x et y les coordonnées, relatives aux bits de poids faible, de l'élément d'image dans la maille acquise, l'interpolation consiste à déterminer DX DY les déplacements à appliquer à la position de l'évènement situé à l'intérieur d'une maille afin de la corriger. Cette correction dépend de x et de y et de la manière dont la maille acquise a été transformée pour devenir la maille corrigée. Si on appelle A,B, C, et D, les sommets d'une maille carrée dans l'image acquise, ces sommets avec la correction de distorsion doivent respectivement être transformés c'est à dire subir des corrections de coordonnées DXa-DYa, DXb-DYb, DXc-DYc et DXd-DYd selon les deux axes de référence de l'image. Ces corrections de coordonnées sont stockées dans une mémoire dite de correction.

La correction de distorsion de la position de l'évènement d'image à replacer est alors donnée par les formules suivantes :

DX = (1-x-y+xy) DXa + (x-xy) DXb + (y-xy) DXc + (xy) DXd

DY = (1-x-y+xy) DYa + (x-xy) DYb + (y-xy) DYc + (xy) DYd

En fait la méthode classique de calcul d'interpolation passe, pour le calcul de chaque déplacement DX ou DY, par deux niveaux de calculs intermédiaires. On calcule d'abord une première correction CI à partir des coordonnées x (ou respectivement y) et des corrections de coordonnées des deux sommets de la maille : DXa (ou DYa) et DXb (ou DYb). Avec deux accès à la mémoire où sont enregistrées les coordonnées des sommets des mailles corrigées on obtient ainsi un résultat intermédiaire CIx (ou CIy). On calcule ensuite une autre correction CJ par interpolation entre les deux autres sommets C et D de la maille. On obtient de la même façon CJx (ou CJy) avec deux autres accès à la mémoire. Le calcul final consiste, à partir de CI et de CJ à obtenir le déplacement DX (ou DY). On a donc, pour obtenir chacun des déplacements d'image d'un l'évènement d'image dans l'image corrigée, procédé à quatre accès mémoire (en A, B, C et D), à quatre multiplications en tenant compte des coordonnées x (ou y), à deux additions pour déterminer CI et CJ, à deux accès aux valeurs intermédiaires CI et CJ, à deux autres multiplications en tenant compte des coordonnées y cette fois (ou x) et enfin à une addition pour obtenir le déplacement final DX (ou DY). Ces nombreuses opérations nuisent à la rapidité de la présentation des images corrigées.

Lorsque le nombre de bits de poids faible à interpoler est peu important, par exemple trois bits, il est possible de précalculer pour chaque maille corrigée les corrections définitives qu'il faut attribuer à des évènements d'image placés à l'intérieur des mailles acquises. On procède ensuite à un adressage direct pour obtenir les valeurs pré-interpolées. Comme la détermination de la maille corrigée a elle-même été obtenue par adressage, cette technique revient à effectuer un adressage direct de la totalité de la correction. Cette technique présente cependant un inconvénient : elle est inexploitable si le nombre de bits d'adressage est trop important. Ceci signifie qu'elle est inexploitable si la précision de la résolution attendue de l'image est trop grande ou, ce qui revient au même, si l'interpolation doit être pratiquée sur un nombre de bits plus grand. En effet lorsque les coordonnés sont données sur 12 bits tout procédé de correction d'image basé en définitive sur une telle technique d'adressage direct, nécessite une capacité mémoire exorbitante. En particulier pour une image à 4096 X 4096 points d'image, il faudrait une mémoire de 16 Méga mots de 24 bits (12 bits pour déterminer X corrigé, et 12 bits pour déterminer Y corrigé). Cette mémoire serait adressée par les X et Y acquis et codés chacun sur 12 bits. Cette capcité dépasse les limites technologiques actuellement accessibles.

Une demande de brevet GB-A-2 026 811 décrit une méthode et une machine pour reproduire une image couleur, un pinceau lumineux effectuant le balayage de l'image originale. Le signal image analogique ainsi obtenu est séparé en n signaux images élémentaires qui sont alors digitalisés, l'un de ces signaux est alors stocké, dans sa totalité, dans une mémoire, tandis que les autres sont échantillonnés selon des modèles réguliers, les valeurs échantillonnées étant stockées en mémoire. Les signaux images élémentaires à sortie digitale sont obtenus dans ladite mémoire, en lisant le premier signal image élémentaire sans modification, en lisant les autres et en effectuant une interpolation des valeurs mémorisées. Les signaux de sortie sont alors utilisés pour obtenir l'image reproduite désirée. L'interpolation s'effectue selon la méthode définie par le préambule de la revendication 1.

L'invention a pour objet de remédier aux inconvénients définis ci-dessus en proposant un procédé d'interpolation où la capacité mémoire n'est pas particulièrement augmentée mais où par contre le calcul d'interpolation peut être fait en temps réel. Le fait que le calcul d'interpolation puisse être fait en temps réel peut être utilisé dans le domaine vidéo. En effet la représentation des mouvements nécessite l'acquisition et le stockage de nombreuses images. De manière à ne pas trop augmenter les capacités mémoire au moment de ce stockage, il peut être utile, étant donné qu'on n'est intéressé qu'au mouvement, de ne stocker que des images à définition dégradée. Dans certains cas, en particulier dans l'étude des mouvements cardiaques par gamma-caméra, les images obtenues ont dès le départ une faible définition. Cependant, au moment de la visualisation, il peut être nécessaire d'amortir les différences de niveau de luminosité et d'augmenter la résolution de l'image en recréant, à partir des images stockées dans la mémoire en faible nombre (faible résolution) des images à haute résolution (introduction d'évènements d'image intermédiaires dans chaque maille d'événements d'image stockée) et avec un nombre de niveaux de gris enrichi. On ne peut cependant créer des évènements d'images intermédiaires que dans la mesure où on peut leur donner, dans la même gamme de niveaux de gris, des niveaux de gris intermédiaires. L'amortissement des transitions lumineuses ne se produit ainsi que si l'écart de niveau de gris entre éléments d'images voisins dans l'image faiblement définie est important. Le calcul en temps réel autorisé par l'invention permet alors la création "au vol" de telles images haute définition à partir d'images basse définition stockées en grand nombre.

L'invention a donc pour objet un procédé d'interpolation multiple de la valeur d'un point P dans une maille élémentaire à au moins quatre sommets (A, B, C, D), d'une image constituée de pixels, des coefficients pondérateurs étant associés à ces sommets, dans lequel :

- les coefficients pondérateurs des pixels de l'image sont stockées dans une mémoire qui est adressée par les bits de poids fort des coordonnées X et Y du point P,
- les coefficients pondérateurs en ces sommets (A, B, C, D) de la maille sont lus simultanément de cette mémoire,
- les coefficients pondérateurs lus sont multipliés par des valeurs précalculées emmagasinées dans une table,
- ces valeurs précalculées sont des contributions (Ka, Kb, Kc, Kd) d'interpolations relatives à tous les lieux de la maille (A, B, C, D), pour une finesse d'interpolation déterminée (x,y), précalculées selon 1-x-y+xy, x-xy, y-yx et xy,
- on attribue au point P comme valeur interpolée le résultat d'une composition algébrique de ces contributions et des coefficients pondérateurs des sommets de la maille, caractérisé en ce que :
- la table emmagasinée en mémoire contient comme valeurs précalculées le produit des coefficients pondérateurs des sommets et de leurs contributions d'interpolation correspondantes.

L'invention sera mieux comprise à la lecture de la description qui suit et à l'examen des figures qui l'accompagnent. Celles-ci ne sont données qu'à titre indicatif et nullement limitatif de l'invention. Les figures montrent

- figure 1 : une représentation schématique fonctionnelle d'un pocédé d'interpolation;
- figure 2 : un schéma fonctionnel des moyens permettant de comprendre le procédé de l'invention;
- figure 3 : une réalisation préférée de l'invention dans une application de type vidéo.

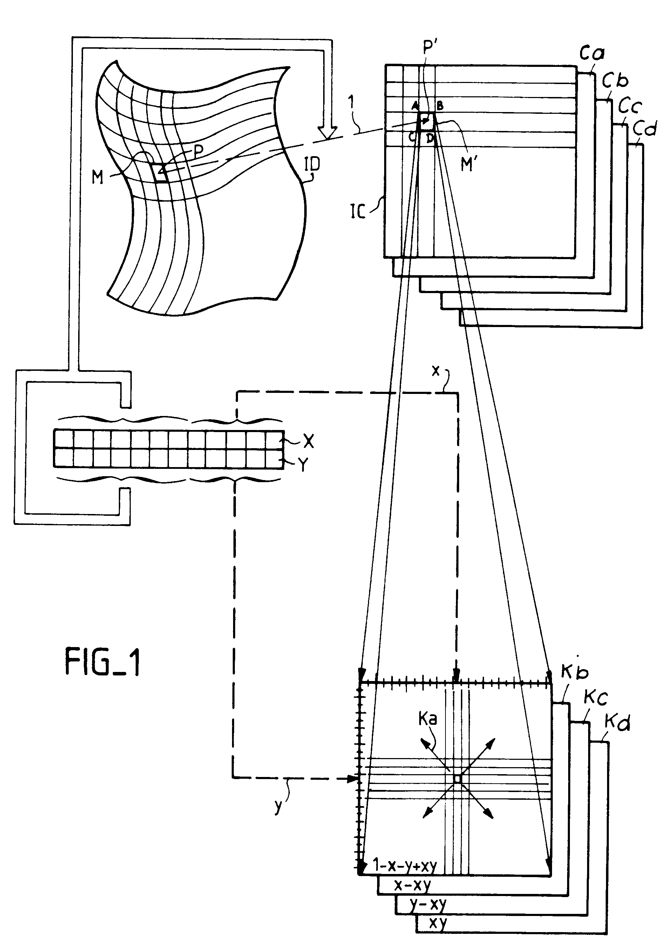

La figure 1 représente schématiquement un procédé d'interpolation semblable à celui utilisé selon l'invention. Un point P, ou événement d'image, est défini, après une acquisition, par ses coordonnées X et Y. Dans un exemple préféré, l'acquisition est une acquisition obtenue à la suite d'une expérimentation avec une gamma-caméra et, pour fixer les idées, les coordonnées X et Y sont données sous forme binaire avec 12 bits de définition. Le point P appartient à une image distordue ID qui est inexploitable. Pour pouvoir être utilisée cette image doit être corrigée par un procédé de correction de distorsion d'image. En définitive, il faut calculer les coordonnées d'un point P' dans une image corrigée IC. Dans les procédés de correction de distorsion on attribue à chaque maille élémentaire M de l'image distordue ID acquise une maille corrigée M' dont les sommets A, B, C et D sont définis par des corrections de coordonnées, dits aussi ici coefficients pondérateurs, DXa - DYa, DXb... DYd. En déterminant la résolution du maillage de correction il est possible d'effectuer une correction de distorsion en deux temps. Un premier temps consiste à attribuer à une maille M une maille M'. Le second temps consiste à interpoler la position du point P' dans la maille M' en fonction de la position du point P dans la maille M et en fonction des coefficients pondérateurs des sommets de la maille. La flèche 1 représente la première opération. Pour la mener à bien on utilise les bits de poids fort des coordonnées X et Y. Dans une réalisation particulière on utilise les sept bits de poids fort de manière à définir un maillage de 128 X 128 mailles dans les images distordues ID et corrigées IC.

On détermine la finesse d'interpolation à obtenir. Dans l'exemple présenté, la finesse d'interpolation à obtenir concerne les cinq bits de poids faible, notés x et y, des coordonnées X et Y. Si on appelle Ka la contribution (1-x-y+xy), Kb la contribution (x-xy), Kc la contribution (y-xy), et Kd la contribution (xy), l'invention a pour but de précalculer les contributions de Ka, Kb, Kc, et Kd, pour toutes les valeurs possibles de x et y correspondant à la finesse d'interpolation à obtenir. On stocke ensuite ces valeurs dans des mémoires mortes appelées matrices d'interpolation. Dans l'exemple cette finesse est mesurée sur cinq bits : chaque coordonnée x ou y peut donc prendre 32 valeurs. En adressant des mémoires préprogrammées contenant les différentes contributions Ka, Kb, Kc et Kd par les coordonnées x et y, on peut obtenir directement les valeur des fonctions représentatives de ces contributions. On détermine ultérieurement DX et DY en appliquant les formules suivantes :

$$DX = Ka.DXa + Kb.DXb + Kc.DXc + Kd.DXd$$
$$DY = Ka.DYa + Kb.DYb + Kc.DYb + Kd.DYd$$

Puisque x et y sont ici codés sur cinq bits chacun, chaque mémoire morte Ka, Kb, Kc et Kd comporte 1024 cases mémoire. Dans la pratique chaque case mémoire comporte une contribution codées sur huit bits. Il y a quatre matrices d'interpolation. Autrement dit l'interpolation nécessite une augmentation mémoire de 4 K octets pour stocker l'ensemble des contributions relatives. Compte tenu du fait que le maillage de correction comporte huit zones mémoires dites de correction de coordonnées et relatives aux déplacements des sommets des mailles : DXa, DYa, DXb,... DYd, du fait que ces corrections de coordonnées sont codées chacune sur 11 bits (un bit de signe, huit bits significatifs et deux bits fractionnaires), et du fait que le maillage de correction est relatif à une résolution 128 X 128, la taille mémoire globale est de 2 fois 16 K mots de 11 bits de mémoire vive, et de 4 K octets de mémoire morte. En effet les corrections de coordonnées doivent pouvoir changer, en fonction du vieillissement de la gamma-caméra par exemple, alors que les contributions relatives précalculées sont immuables. Elles peuvent donc être stockées dans une mémoire morte.

La taille mémoire supplémentaire pour les matrices d'interpolation est par conséquent négligeable. Dans l'application il est utile de remarquer que le précalcul des contributions d'interpolation peut être simplifié en utilisant, pour la programmation des mémoires Ka, Kb et Kc les résultats obtenus pour le précalcul de la mémoire Kd. La multiplication x.y n'a pas être refaite.

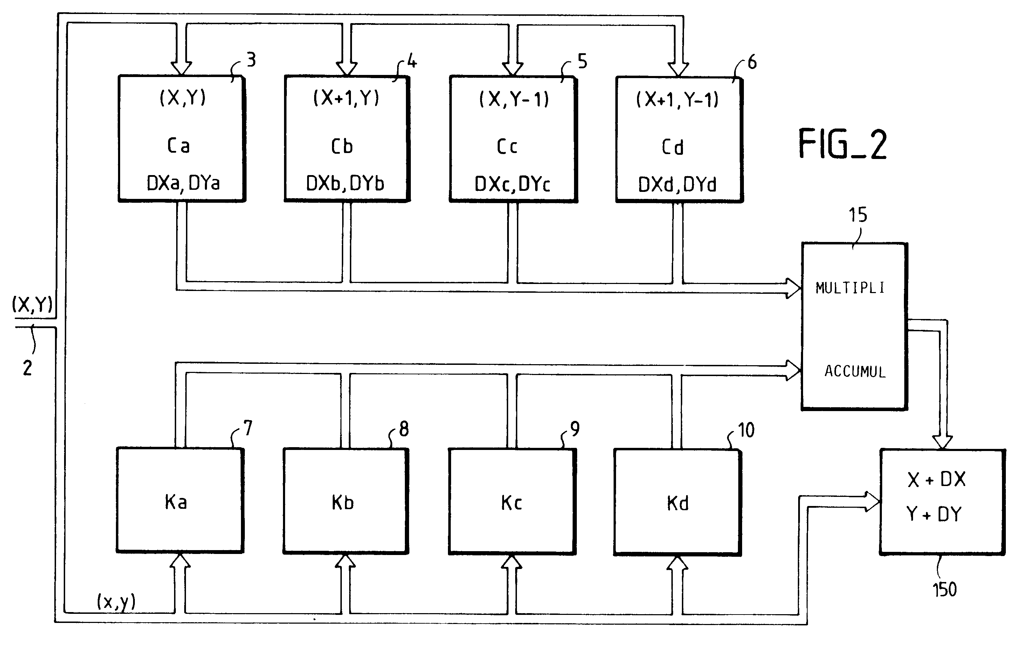

La figure 2 représente le mode préféré de détermination des coordonnées corrigées des point P' dans la maille M'. Un bus 2 achemine les coordonnées X et Y jusqu'aux mémoires de corrections de coordonnées 3 à 6 d'une part, et jusqu'aux mémoires d'interpolation 7 à 10 d'autre part. Un multiplicateur-accumulateur 15 relié aux sorties de ces mémoires reçoit ensuite des mémoires de corrections, les corrections de coordonnées DX (ou DY) d'une part, et des mémoires d'interpolation 7 à 10 les contributions relatives d'interpolation K d'autre part. Il effectue les multiplications correspondantes et l'accumulation des résultats de ces multiplications. IL délivre selon le cas le déplacement DX puis le déplacement DY. Avec le multiplicateur accumulateur 15 le traitement est effectué en série. Un circuit de correction 150 délivre ensuite les coordonnées corrigées des évènements d'image en additionnant aux coordonnées distordues les déplacements calculés.

Ajouté au fait que la taille mémoire a ainsi peu augmenté le procédé de l'invention peut aussi permettre de plus de gagner du temps. En effet l'adressage des corrections de coordonnées des différents sommets de la maille de correction M' peut être simultané. En effet X et Y, ou du moins les 7 bits de poids fort de X et Y, représentent le sommet A de la maille. Les sommets B, C et D s'en déduisent par une addition ou une soustraction d'une unité de bit de poids le plus faible parmi les bits de poids fort. En conséquence on peut avoir accès simultanément aux 8 coefficients pondérateurs DXa, DYa,etc. Dans le même temps il est également possible d'accéder simultanément aux contributions Ka, Kb, Kc et Kd. En remplaçant alors le multiplicateur-accumulateur 15 par un jeu de quatre multiplicateurs affectés respectivement aux mémoires 3 et 7, 4 et 8, 5 et 9 et 6 et 10, et en les reliant à un additionneur on peut accélérer les calculs des déplacements. En effet lors d'un cycle d'horloge suivant celui où les mémoires ont été adressées il est possible au moyen des multiplicateurs d'effectuer chacune des multiplications K.DX (ou K.DY). Au troisième temps de cycle on dispose des valeurs de déplacement DX (ou DY). Trois temps de cycles encore plus tard on dispose de l'autre valeur de déplacement DY (ou DX). Il est même possible de disposer de ces deux valeurs de déplacement simultanément en doublant encore le nombre des multiplicateurs.

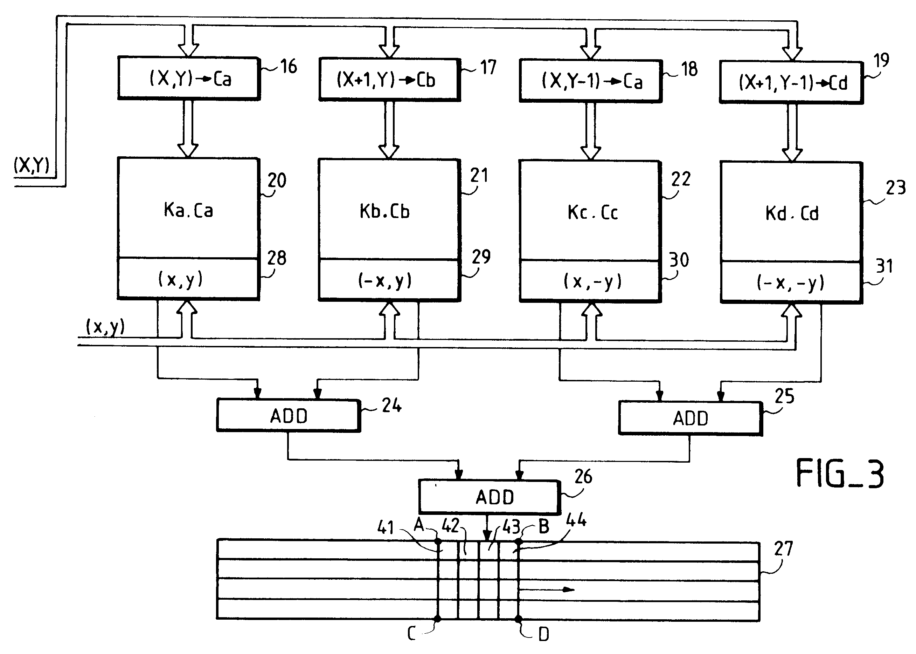

L'application vidéo représentée en partie sur la figure 3, est semblable. Dans celle-ci une trame d'une image disponible a une faible résolution. Par exemple elle comporte 64 lignes et 64 colonnes : il y a 4 K positions possibles des évènements d'image et dans un exemple chaque élément d'images peut être codé sur 8 bits soit 256 niveaux de gris, de luminance ou de chrominance. Le stockage des images est donc peu exigeant. La reconstruction, au vol, d'une image plus finement définie, par exemple avec un pas de 256 X 256 rend nécessaire le calcul du niveau de gris en 16 lieux d'une maille du maillage 64 X 64. Autrement dit une maille repérée par quatre sommets adjacents appartenant à deux lignes adjacentes de l'image stockée doit être morcelée en 4 x 4 = 16 parties. La coordonnée x codée sur 2 bits doit pouvoir valoir de 1 à 4 et la coordonnée y aussi codée sur 2 bits doit pouvoir prendre également les valeurs de 1 à 4. Connaissant le niveau de gris, le coefficient pondérateur, Ca, Cb, Cc et Cd en chacun des sommets A, B, C et D de la maille, il est possible, par un calcul similaire à celui présenté plus haut de calculer les niveaux de gris intermédiaires des points intermédiaires contenus dans la maille.

Selon l'invention on s'est cependant rendu compte que, plutôt que de n'effectuer que le calcul des contributions Ka, Kb,Kc et Kd, il était ici préférable et possible d'effectuer le précalcul de toutes les multiplications nécessaires. En effet, étant donné que les niveaux de gris ne sont donnés pour chaque point que sur huit bits (256 niveaux), il est possible de remplacer les mémoires d'interpolation Ka, Kb, Kc et Kd à 16 cases mémoires (16 positions possibles dans la maille) par des mémoires d'interpolation composites à 16x256 cases mémoires. Les précalculs effectués dans ces mémoires composites sont effectués sur 8 bits.

Les transitions brutales de niveau de gris entre des éléments d'image voisins de l'image faiblement définie sont alors adoucies par l'introduction d'éléments d'image intermédiaires, dont les niveaux de gris sont fixés à des niveaux intermédiaires dans la même gamme de niveaux de gris, à ceux de ces éléments d'image voisins.

Dans une première phase de ce traitement rapide, des images sources de faible définition sont lues à chaque temps de cycle pour déterminer les niveaux de gris, ou coefficients pondérateurs, Ca à Cd des quatre sommets A à D d'une maille. La mémoire source peut être quadruplée de manière à être adressée simultanément en X et Y pour délivrer en même temps les coefficients pondérateurs Ca à Cd. De manière à éviter le coût imposé par le quadruplement de la mémoire on peut l'organiser différemment en séparant les évènements d'image pairs en x et y. Cette organisation différente permet alors de lire simultanément, mais en des zones différentes de cette mémoire, les coefficients pondérateurs Ca à Cd des quatres sommets de la maille. Au besoin un multiplexage de lecture de cette mémoire peut aussi être organisé. La mémoire de l'image source, ou pour simplifier l'explication les mémoires sources 16 à 19, délivrent alors les quatre coefficents pondérateurs sur 8 bits représentatifs chacun du niveau de gris d'un des sommets de la maille. Ces niveaux de gris peuvent être utilisés comme entrée d'adresse de chacune des mémoires 20 à 23 de précalcul composite ayant effectué le produit K.C,et recevant comme autre adresse des codes correspondant successivement à chacune des quatre positions intermédiaires des points interpolés 41 à 44 situés sur une même ligne dans la maille. Dans une ligne y est fixé, seul x varie (de 1 à 4). Les mémoires composites 20 à 23 délivrent alors chacune successivement quatre résultats correspondant au calcul de la luminosité pondérée en quatre lieux dans la maille en fonction d'un des sommets de cette maille. Pour chaque lieu un jeu d'additionneurs 24 à 26 permet d'additionner les résultats délivrés par les quatre mémoires 20 à 23 et de délivrer la luminosité pondérée correspondant à ce point. Avec un temps de cycle de lecture de 166 nomosecondes, les quatres luminosités peuvent être interpolées en 664 nomosecondes. Les luminosités en 256 points d'une ligne vidéo peuvent donc être ainsi calculées en 43 microsecondes. La visualisation de l'image peut alors être obtenue en mode vidéo classique. En effet, en lecture vidéo classique à 25 images par seconde, la lecture d'une ligne d'une image dure 64 microsecondes. La compatibilité de ces deux durées (64 microsecondes étant supérieur à 43 microsecondes) qualifie le procédé d'interpolation ainsi présenté pour la visualisation "au vol" d'images faiblement définies en des images hautement définies.

Le précalcul des mémoires composites présente de plus une particularité. Si on accepte de coter x = O et y = O le centre d'une maille, on se rend compte que la contribution composite attribuée à un point peut être simplifiée. Il suffit de ne calculer qu'une seule mémoire composite et de la réaliser en quatre exemplaires. Seules les valeurs d'adressage utilisées pour accéder à ces mémoires composites changent. Selon la contribution composite envisagée, selon le sommet, on fera subir aux adresses x et y des inversions de valeurs -x et -y. Cette manière d'agir, présente un intérêt. Lors de la fabrication il n'y a pas lieu de se préoccuper d'une différentiation quelconque entre les mémoires 20 à 23. Seul des décodeurs respectifs d'accès 28 à 31 sont ainsi particularisés en fonction de leur objet.

Le mode d'interpolation préconisé dans ces deux applications est de préférence un mode linéaire. Compte tenu du fait qu'on cherche à représenter des images à deux dimensions le mode d'interpolation sera même bilinéaire. Cependant l'invention peut être transposée à une interpolation d'un point dans un espace géométrique à trois dimensions, ou même dans un espace théorique à M dimensions. En outre l'interpolation n'est pas nécessairement du type linéaire. D'autres fonctions que la fonction linéaire sont aussi envisageables. S'agissant d'une maille à N sommets, il peut seulement être nécessaire que la somme des contributions relatives à chacun de ces sommets soit égale à 1.

En outre la définition d'une maille à N sommets ne doit pas être comprise uniquement dans le cas où N vaut quatre comme ce qui vient d'être décrit. Dans le cas où la maille à une forme différente, par exemple triangulaire ou hexagonale, le nombre de sommets peut être différent. Dans ces derniers cas, plutôt que de calculer les contributions selon une définition cartésienne, il peut s'avérer préférable d'utiliser une définition polaire.

**Revendications**

1. Procédé d' interpolation multiple de la valeur d'un point P dans une maille élémentaire à au moins quatre sommets (A, B, C, D), d'une image constituée de pixels, des coefficients pondérateurs (Ca ... Cd) étant associés à ces sommets, dans lequel :
   - les coefficients pondérateurs des pixels de l'image sont stockées dans une mémoire (16-19) qui est adressée par les bits de poids (X,Y) fort des coordonnées X et Y du point P,
   - les coefficients pondérateurs en ces sommets (A, B, C, D) de la maille sont lus simultanément de cette mémoire,

- les coefficients pondérateurs lus sont multipliés par des valeurs précalculées (Ka ... Kd) emmagasinées dans une table (20-23),
- ces valeurs précalculées sont des contributions (Ka, Kb, Kc, Kd) d' interpolations relatives à tous les lieux de la maille (A, B, C, D), pour une finesse d'interpolation déterminée (x,y), précalculées selon $Ka = 1-x-y+xy$, $Kb = x-xy$, $Kc = y-yx$ et $Kd = xy$,
- on attribue au point P comme valeur interpolée le résultat d'une composition algébrique de ces contributions et des coefficients pondérateurs des sommets de la maille, caractérisé en ce que
- la table emmagasinée en mémoire (20-23) contient comme valeurs précalculées le produit des coefficients pondérateurs (Ca ... Cd) des sommets et de leurs contributions d'interpolation (Ka ... Kd) correspondantes.

2. Procédé selon la revendication 1, caractérisé en ce que l'interpolation multiple est une interpolation double (en x et y).

3. Procédé selon la revendication 2, caractérisé en ce que l'interpolation double est une interpolation bilinéaire.

4. procédé selon l'une quelconque des revendications 1 à 3, caractérisé en ce que la valeur représente la luminance ou la chrominance d'un élément d'image.

5. Procédé selon la revendication 4, caractérisé en ce qu'on précalcule (20, 30) en partie les valeurs interpolées en fonction de valeurs prédéterminées des coefficients pondérateurs.

6. Procédé selon l'une quelconque des revendication 1 à 5, caractérisé en ce qu'il est appliqué à une image de type vidéo.

## Claims

1. Process for the multiple interpolation of the value of a point P in an elementary mesh having at least four nodes (A, B, C, D) of an image constituted by pixels, weighting coefficients (Ca...Cd) being associated with said nodes, wherein the weighting coefficients of the pixels of the image are stored in a memory (16-19), which is addressed by the most significant bits ((x, y) of the coordinates X and Y of the point P, the weighting coefficient in said nodes (A, B, C, D) of the mesh are simultaneously read from said memory, the read weighting coefficients are multiplied by the precalculated values (Ka...Kd) stored in a table (20-23), said precalculated values are the contributions (Ka, Kb, Kc, Kd) of interpolations relative to all the locations of the mesh (A, B, C, D) for a given interpolation fineness (x, y) precalculated according to $Ka = 1-x-y+xy$, $Kb = x-xy$, $Kc = y-yx$ and $Kd = xy$, and to the point P is attributed as the interpolated value the result of an algebraic composition of these contributions and the weighting coefficients of the nodes of the mesh, characterized in that the table (20-23) stored in the memory contains as precalculated values the product of the weighting coefficients (Ca...Cd) of the nodes and of their corresponding interpolation contributions (Ka...Kd).

2. Process according to claim 1, characterized in that the multiple interpolation is a double interpolation (in x and y).

3. Process according to claim 2, characterized in that the double interpolation is a bilinear interpolation.

4. Process according to any one of the claims 1 to 3, characterized in that the value represents the luminescence or chrominance of an image element.

5. Process according to claim 4, characterized in that a partial precalculation (20, 30) takes place of the interpolated values as a function of predetermined values of the weighting coefficients.

6. Process according to any one of the claims 1 to 5, characterized in that it is applied to a video-type image.

## Patentansprüche

1. Multiples Interpolationsverfahren des Wertes eines Punktes P in einem Elementargitter mit wenigstens 4 Spitzen (A, B, C, D) eines aus Pixeln bestehenden Bildes, wobei Gewichtungskoeffizienten (Ca...Cd) diesen Spitzen zugeordnet sind, bei dem:
- die Gewichtungskoeffizienten der Pixel des Bildes gespeichert werden in einem Speicher (16-19), der adressiert wird durch die Bits hoher Wertigkeit (X, Y) der Koordinaten X und Y des Punkts P,
- die Gewichtungskoeffizienten in diesen Spitzen (A, B, C, D) des Gitters simultan aus diesem Speicher gelesen werden,

- die ausgelesenen Gewichtungskoeffizienten mit vorberechneten, in einer Tabelle (20-23) gespeicherten Werten (Ka...Kd) multipliziert werden,

- diese vorberechneten Werte Interpolationskontributionen (Ka, Kb, Kc, Kd) bezüglich aller Orte des Gitters (A, B, C, D) sind, für eine bestimmte Interpolationsfeinheit (x, y), vorberechnet gemäß Ka = 1-x-y + xy, Kb = x-xy, Kc = y-yx und Kd = xy,

- man dem Punkt P als interpolierten Wert das Resultat einer algebraischen Komposition dieser Kontributionen und Gewichtungskoeffizienten der Spitzen des Gitters zuweist,
dadurch **gekennzeichnet**, daß

- die gespeicherte Tabelle (20-23) als vorberechnete Werte das Produkt der Gewichtungskoeffizienten (Ca...Cd) der Spitzen und ihrer entsprechenden Interpolationskontributionen (Ka...Kd) enthält.

2. Verfahren nach Anspruch 1, dadurch gekennzeichnet, daß die multiple Interpolation eine doppelte Interpolation (in x und y) ist.

3. Verfahren nach Anspruch 2, dadurch gekennzeichnet, daß die doppelte Interpolation eine bilineare Interpolation ist.

4. Verfahren nach einem der Ansprüche 1 bis 3, dadurch gekennzeichnet, daß der Wert die Helligkeit oder den Farbton eines Bildelements darstellt.

5. Verfahren nach Anspruch 4, dadurch gekennzeichnet, daß man die interpolierten Werte zum Teil auf Grund von vorbestimmten Werten der Gewichtungskoeffizienten vorberechnet (20, 30).

6. Verfahren nach einem der Ansprüche 1 bis 5, dadurch gekennzeichnet, daß es angewandt wird bei einem Bild des Videotyps.

FIG_1

FIG_2

EP 0 308 315 B1

EP 0 308 315 B1

| (X,Y)→Ca ⟍16 | (X+1,Y)→Cb ⟍17 | (X,Y-1)→Ca ⟍18 | (X+1,Y-1)→Cd ⟍19 |
|---|---|---|---|

(X,Y)

| Ka.Ca ⟍20 | Kb.Cb ⟍21 | Kc.Cc ⟍22 | Kd.Cd ⟍23 |
|---|---|---|---|
| (x,y) ⟍28 | (-x,y) ⟍29 | (x,-y) ⟍30 | (-x,-y) ⟍31 |

(x,y)

| ADD ⟍24 | ADD ⟍25 |
|---|---|

| ADD ⟍26 |
|---|

FIG_3

41 A 42 43 B 44

27

C D